(19) Europäisches Patentamt
European Patent Office
Office européen des brevets

(11) EP 4 101 449 A1

(12) **EUROPEAN PATENT APPLICATION**
published in accordance with Art. 153(4) EPC

(43) Date of publication:
**14.12.2022 Bulletin 2022/50**

(21) Application number: **20915529.0**

(22) Date of filing: **15.07.2020**

(51) International Patent Classification (IPC):
***A61K 31/426*** *(2006.01)* ***A61P 31/12*** *(2006.01)*
***A61P 31/14*** *(2006.01)*

(86) International application number:
**PCT/CN2020/102143**

(87) International publication number:
**WO 2021/147273 (29.07.2021 Gazette 2021/30)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **21.01.2020 CN 202010071688**

(71) Applicant: **Academy of Military Medical Sciences**
**Beijing 100850 (CN)**

(72) Inventors:
- **ZHONG, Wu**
**Beijing 100850 (CN)**
- **XIAO, Gengfu**
**Beijing 100850 (CN)**
- **HU, Zhihong**
**Beijing 100850 (CN)**
- **WANG, Manli**
**Beijing 100850 (CN)**
- **CAO, Ruiyuan**
**Beijing 100850 (CN)**
- **ZHANG, Leike**
**Beijing 100850 (CN)**
- **LI, Wei**
**Beijing 100850 (CN)**
- **LI, Yuexiang**
**Beijing 100850 (CN)**
- **ZHAO, Lei**
**Beijing 100850 (CN)**
- **LI, Song**
**Beijing 100850 (CN)**

(74) Representative: **Böhm, Brigitte**
**Weickmann & Weickmann**
**Patent- und Rechtsanwälte PartmbB**
**Postfach 860 820**
**81635 München (DE)**

(54) **APPLICATION OF NITAZOXANIDE AND ACTIVE FORM THEREOF, TIZOXANIDE, IN TREATMENT OF SARS-COV-2 INFECTION**

(57) Nitazoxanide represented by formula I and an active form thereof, tizoxanide compound, represented by formula II, a geometric isomer thereof and pharmaceutically acceptable salt thereof and/or solvate thereof and/or hydrate thereof, and a pharmaceutical composition containing this compound, used for preventing and/or treating coronavirus (such as SARS-CoV-2) infection.

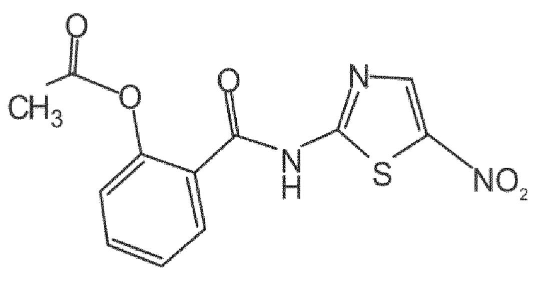

Formular I

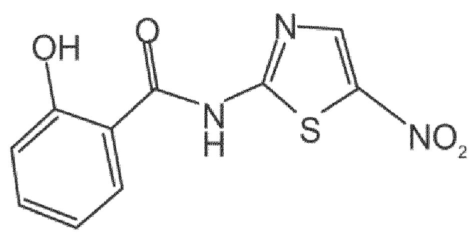

Formular II

EP 4 101 449 A1

## Description

The invention is based on and claims the benefit of priority from Chinese application No. 202010071688.8, filed on January 21, 2020, the disclosure of which are incorporated herein by reference in its entirety.

## Technical Field

The present application relates to use of nitazoxanide represented by Formula I and its active form tizoxanide represented by Formula II, a geometric isomer, a pharmaceutically acceptable salt and/or a solvate and/or a hydrate thereof and a pharmaceutical composition comprising the above-mentioned compound in the treatment of a coronavirus infection, especially a SARS-CoV-2 infection.

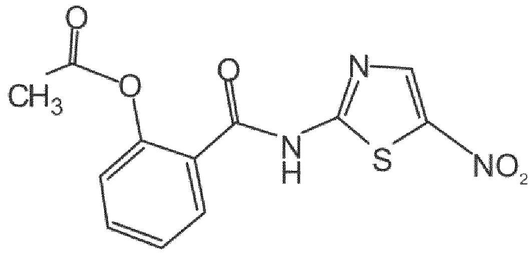

Formular I

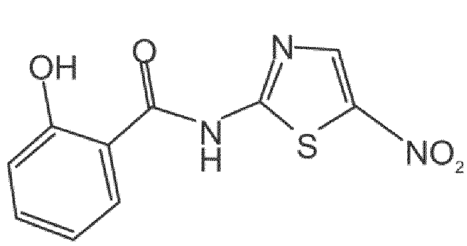

Formular II

## Background Art

Nitazoxanide (compound of formula I), which has a chemical name of 2-(acetyloxy)-N-(5-nitro-2-thiazolyl) benzamide) , is a thiazole drug, and first synthesized in the 1970s (Rossignol, J.F., Cavier, R., 1975. Synthesis and antiparasitic activity of 2-benzamidonitrothiazoles. Chem. Abstr. 83, 28216n.). Nitazoxanide is a prodrug, which is quickly hydrolyzed to its active metabolite tizoxanide (TIZ, compound of formula II) *in vivo* after administration to exert its therapeutic effect.

Initially, nitazoxanide was developed as an oral antiparasitic agent, and has good antiprotozoal activity *in vitro* and in different animal models. It was first studied in humans for treatment of intestinal cestodes. Later, it was found having a good inhibitory activity against emerging protozoa and Cryptosporidium. In 1998, the FDA approved nitazoxanide as a clinical drug for treatment of infection caused by Cryptosporidium parvum and Giardia in adults and children at least 12 months of age. In 2006, Clinical trials have indicated nitazoxanide can effectively treat diarrhea in children caused by rotavirus infection. In addition, nitazoxanide has also been confirmed to have good clinical therapeutic effect on viral gastroenteritis and hepatitis C in adults (Rossignol JF, Nitazoxanide: a first-in-class broad-spectrum antiviral agent, Antiviral Res. 2014;110:94-103.).

In recent years, several laboratory studies have confirmed that nitazoxanide has broad-spectrum antiviral activity. A series of laboratory studies have shown that nitazoxanide can block maturation of the hemagglutinin of influenza virus at the post-translational stage, has highly effective inhibitory activity against influenza A and influenza B. Clinical trial results show that administration of nitazoxanide 600 mg twice daily for five days reduced the duration of common influenza. (Haffizulla J, Hartman A, Hoppers M, et al, Effect of nitazoxanide in adults and adolescents with acute uncomplicated influenza: a double-blind, randomised, placebo-controlled, phase 2b/3 trial, Lancet Infect Dis. 2014;14(7):609-18.).

The 2019 novel coronavirus (2019-nCoV) is a new coronavirus strain that has never been found in humans before. On February 11, 2020, the International Committee on Taxonomy Viruses (ICTV) announced that the official name of 2019 novel Coronavirus (2019-nCoV) is called severe acute respiratory syndrome coronavirus 2 (SARS-CoV-2). On the same day, the World Health Organization (WHO) announced that the official name of the disease caused by this virus is COVID-19. The symptoms of SARS-CoV-2 virus infection are mainly pneumonia, and can be divided into simple infection, mild pneumonia, severe pneumonia, acute respiratory distress syndrome, sepsis, septic shock and so on according to the severity of disease. Patients with simple infection may have non-specific symptoms, such as fever, cough, sore throat, nasal congestion, fatigue, headache, muscle pain or discomfort, and the elderly and immunosuppressed people may have atypical symptoms. Patients with mild pneumonia mainly have cough, dyspnea and polypnea. Severe pneumonia can be seen in adolescents, adults or children, and the main symptoms of which include increased breathing frequency, severe respiratory failure or dyspnea, central cyanosis, drowsiness, unconsciousness or convulsion, gasp, etc. The lung images of acute respiratory distress syndrome are bilateral ground glass shadows, which cannot

be completely explained by effusion, lobar exudation or atelectasis or lung mass shadows, and the main symptom of which is pulmonary edema. Patients with sepsis often have fatal organ dysfunction, and the most critical patients are those with septic shock, and they may have a high probability of death.

At present, the SARS-CoV-2 infection is mainly treated with supportive therapy in clinic, and no specific antiviral drug is available.

**Contents of the Invention**

The purpose of the present application is to discover a drug with an antiviral activity against SARS-CoV-2 for the treatment of SARS-CoV-2 infection. Through creative research in the present application, it is found that nitazoxanide represented by Formula I has a function of protection of cells infected by SARS-CoV-2 and inhibiting the replication of SARS-CoV-2, and has a good potential therapeutic effect in the treatment of a disease caused by a SARS-CoV-2.

The present application relates to a thiazole compound represented by Formula I or Formular II, a geometric isomer, a pharmaceutically acceptable salt and/or a solvate or a hydrate thereof:

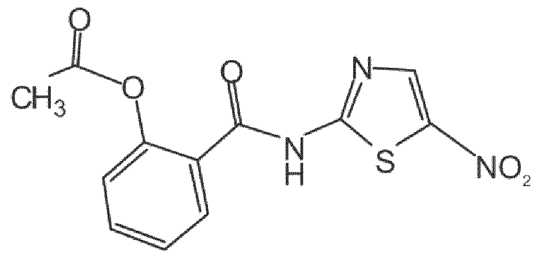

Formular I

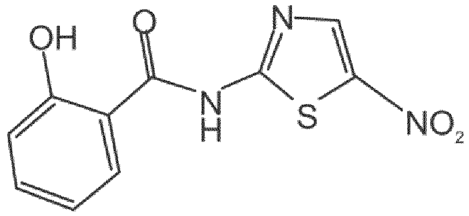

Formular II

In some embodiments, the pharmaceutically acceptable salts of the compound of the present application include an inorganic or organic acid salt thereof and an inorganic or organic base salt thereof. The present application relates to all forms of the above salts, including but not limited to: sodium salt, potassium salt, calcium salt, lithium salt, meglumine salt, hydrochloride salt, hydrobromide salt, hydroiodide salt, nitrate salt, sulfate, hydrogen sulfate, phosphate, hydrogen phosphate, acetate, propionate, butyrate, oxalate, pivalate, adipate, alginate, lactate, citrate, tartrate, succinate, maleate, fumarate, picrate, aspartate, gluconate, benzoate, methanesulfonate, ethanesulfonate, benzenesulfonate, p-toluenesulfonate and embonate and so on.

According to the present application, the compound represented by Formula I or Formular II can protect cells from cytopathic effect (CPE) caused by SARS-CoV-2 virus infection, inhibit the replication of SARS-CoV-2 in a cell and reduce the nucleic acid load of SARS-CoV-2 in a cell culture.

After creative invention and research, the inventors of the present application have discovered some new features of the compound represented by Formula I or Formular II: the compound represented by Formula I or Formular II can reduce the viral nucleic acid load in SARS-CoV-2 infected cells at micromolar concentration level.

The present application also relates to use of the compound represented by Formula I and/or Formular II, a geometric isomer, a pharmaceutically acceptable salt and/or a solvate and/or a hydrate thereof in the manufacture of a medicament for the prevention and/or the treatment of a disease or an infection caused by a coronavirus (such as SARS-CoV-2) (including but not limited to a respiratory disease (e.g., simple infection (such as fever, cough and sore throat), pneumonia, acute respiratory infection or severe acute respiratory infection, hypoxic respiratory failure and acute respiratory distress syndrome), sepsis and septic shock, etc.),

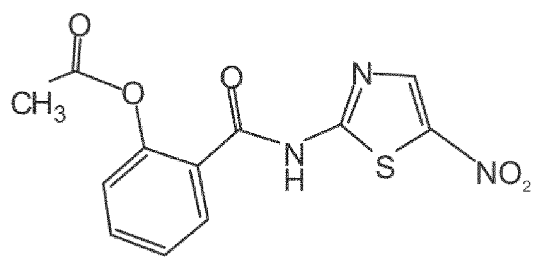

Formular I

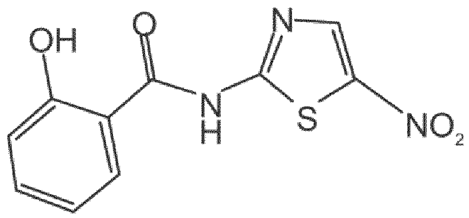

Formular II.

The present application also relates to use of the compound represented by Formula I and/or Formular II, a geometric isomer, a pharmaceutically acceptable salt and/or a solvate and/or a hydrate thereof in the manufacture of a medicament as a coronavirus (such as SARS-CoV-2) inhibitor.

The present application also relates to use of the compound represented by Formula I and/or Formular II, a geometric isomer, a pharmaceutically acceptable salt and/or a solvate and/or a hydrate thereof in the manufacture of a medicament for inhibiting the replication or reproduction of coronavirus (such as SARS-CoV-2) in a cell (e.g., a cell of mammal).

The present application also relates to a pharmaceutical composition, which comprises the compound represented by Formula I and/or Formular II, a geometric isomer, a pharmaceutically acceptable salt and/or a solvate and/or a hydrate thereof,
preferably, the pharmaceutical composition further comprises a pharmaceutically acceptable carrier or excipient; specifically, the pharmaceutical composition is a solid preparation, an injection, an external preparation, a spray, a liquid preparation, or a compound preparation.

The present application also relates to use of the pharmaceutical composition comprising the compound represented by Formula I and/or Formular II, a geometric isomer, a pharmaceutically acceptable salt and/or a solvate and/or a hydrate thereof or the compound represented by Formula I and/or Formular II, a geometric isomer, a pharmaceutically acceptable salt and/or a solvate and/or a hydrate thereof in the manufacture of a medicament for the prevention and/or the treatment of a disease including but not limited to a respiratory disease (simple infection (such as fever, cough and sore throat), pneumonia, acute respiratory infection or severe acute respiratory infection, hypoxic respiratory failure and acute respiratory distress syndrome), sepsis and septic shock, etc.

The present application also relates to a method for preventing and/or treating a disease in a mammal in need thereof or a method for inhibiting the replication or reproduction of coronavirus (such as SARS-CoV-2) in a mammal in need thereof, wherein the method comprises administering to the mammal in need thereof a therapeutically and/or prophylactically effective amount of a pharmaceutical composition comprising a compound represented by Formula I and/or Formular II, a geometric isomer, a pharmaceutically acceptable salt and/or a solvate and/or a hydrate thereof, or a compound represented by Formula I and/or Formular II, a geometric isomer, a pharmaceutically acceptable salt and/or a solvate and/or a hydrates thereof, wherein the disease includes a disease or an infection caused by a coronavirus (such as SARS-CoV-2) (e.g., severe acute respiratory infection (SARI)).

In some embodiments, the disease is a disease or an infection caused by a SARS-CoV-2.

In some embodiments, the disease caused by a SARS-CoV-2 includes but is not limited to a respiratory disease (e.g. simple infection (such as fever, cough and sore throat), pneumonia, acute respiratory infection or severe acute respiratory infection, hypoxic respiratory failure and acute respiratory distress syndrome), sepsis and septic shock, etc.

The present application also relates to use of the pharmaceutical composition comprising a compound represented by Formula I and/or Formular II, a geometric isomer, a pharmaceutically acceptable salt and/or a solvate and/or a hydrate thereof in the manufacture of a medicament for the prevention and/or the treatment of a disease or an infection caused by a coronavirus (such as SARS-CoV-2) (e.g., a respiratory disease (e.g., simple infection (such as fever, cough and sore throat), pneumonia, acute respiratory infection or severe acute respiratory infection, hypoxic respiratory failure and acute respiratory distress syndrome), sepsis and septic shock, etc.

The present application also relates to use of the pharmaceutical composition comprising a compound represented by Formula I and/or Formular II, a geometric isomer, a pharmaceutically acceptable salt and/or a solvate and/or a hydrate thereof in the manufacture of a medicament as a coronavirus (such as SARS-CoV-2) inhibitor.

The present application also relates to use of the pharmaceutical composition comprising a compound represented by Formula I and/or Formular II, a geometric isomer, a pharmaceutically acceptable salt and/or a solvate and/or a hydrate thereof in the manufacture of a medicament for inhibiting the replication or reproduction of coronavirus (such as SARS-CoV-2) in a cell (e.g., a cell of mammal).

The present application also relates to the compound represented by Formula I and/or Formular II, a geometric isomer, a pharmaceutically acceptable salt and/or a solvate and/or a hydrate thereof, for use in preventing and/or treating a disease or an infection caused by a coronavirus (such as SARS-CoV-2) (including but not limited to a respiratory disease (e.g., simple infection (such as fever, cough and sore throat), pneumonia, acute respiratory infection or severe acute respiratory infection, hypoxic respiratory failure and acute respiratory distress syndrome), sepsis and septic shock, etc.).

The present application also relates to the compound represented by Formula I and/or Formular II, a geometric isomer, a pharmaceutically acceptable salt and/or a solvate and/or a hydrate thereof, for use as a coronavirus (such as SARS-CoV-2) inhibitor.

The present application also relates to the compound represented by Formula I and/or Formular II, a geometric isomer, a pharmaceutically acceptable salt and/or a solvate and/or a hydrate thereof, for use in inhibiting the replication or reproduction of coronavirus (such as SARS-CoV-2) in a cell (e.g., a cell of mammal).

The present application also relates to the pharmaceutical composition comprising a compound represented

by Formula I and/or Formular II, a geometric isomer, a pharmaceutically acceptable salt and/or a solvate and/or a hydrate thereof, for use in preventing and/or treating a disease or an infection caused by a coronavirus (such as SARS-CoV-2) (e.g., a respiratory disease (e.g., simple infection (such as fever, cough and sore throat), pneumonia, acute respiratory infection or severe acute respiratory infection, hypoxic respiratory failure and acute respiratory distress syndrome), sepsis and septic shock, etc.).

The present application also relates to the pharmaceutical composition comprising a compound represented by Formula I and/or Formular II, a geometric isomer, a pharmaceutically acceptable salt and/or a solvate and/or a hydrate thereof, for use as a coronavirus (such as SARS-CoV-2) inhibitor.

The present application also relates to the pharmaceutical composition comprising a compound represented by Formula I and/or Formular II, a geometric isomer, a pharmaceutically acceptable salt and/or a solvate and/or a hydrate thereof, for use in inhibiting the replication or reproduction of coronavirus (such as SARS-CoV-2) in a cell (e.g., a cell of mammal).

In some embodiments, the disease caused by a SARS-CoV-2 described in the present application is COVID-19.

In some embodiments, the mammal of the present application includes bovine, equine, caprid, suidae, canine, feline, rodent, primate, wherein the preferred mammal is a human, a cat, a dog, or a pig.

In the present application, the official name of the term "2019 novel coronavirus (2019-nCoV)" is severe acute respiratory syndrome coronavirus 2 (SARS-CoV-2).

In the present application, the official name of the term "disease caused by 2019 novel coronavirus (2019-nCoV)" is COVID-19.

The pharmaceutical composition described in the present application can be prepared into various forms according to different administration routes.

According to the present application, the pharmaceutical composition can be administered in any one of the following routes: oral administration, spray inhalation, rectal administration, nasal administration, buccal administration, vaginal administration, topical administration, parenteral administration such as subcutaneous, intravenous, intramuscular, intraperitoneal, intrathecal, intraventricular, intrasternal and intracranial injection or infusion, or administration with the help of an explant reservoir. Among them, oral, intraperitoneal or intravenous administration is preferred.

When orally administered, the compound represented by Formular I, a geometric isomer, a pharmaceutically acceptable salt and/or a solvate and/or a hydrate thereof can be prepared into any form of orally acceptable preparation, including but not limited to a tablet, a capsule, an aqueous solution or an aqueous suspension. Generally, the carrier for use in a tablet includes lactose and corn starch, and a lubricant such as magnesium stearate can also be added. The diluent for use in a capsule generally includes lactose and dry corn starch. The aqueous suspension is usually used by mixing an active ingredient with a suitable emulsifier and a suitable suspending agent. If necessary, a sweetener, a flavoring agent or a coloring agent can also be added to the above-mentioned forms of oral preparation.

When rectally administered, the compound represented by Formular I, a geometric isomer, a pharmaceutically acceptable salt and/or a solvate, and/or a hydrate thereof can generally be prepared in a form of suppository, which is prepared by mixing the drug with a suitable non-irritating excipient. The excipient is present in solid state at room temperature, but melts at the rectal temperature to release the drug. Such excipient includes cocoa butter, beeswax and polyethylene glycol.

When topically administered, especially for the treatment of easily accessible affected-surface or organ, such as eye, skin, or lower intestinal neurological disease by topical application, the compound represented by Formular I, a geometric isomer, a pharmaceutically acceptable salt and/or a solvate and/or a hydrate thereof can be prepared in various forms of topical preparations according to different affected-surfaces or organs, the specific instructions are as follows:

When topically administered to eye, the compound represented by Formular I, a geometric isomer, a pharmaceutically acceptable salt and/or a solvate and/or a hydrate thereof can be formulated into a preparation form such as micronized suspension or solution, the carrier used is isotonic sterile saline with a certain pH, and a preservative such as benzyl chloride alkoxide may or may not be added. In addition, for administration to eye, the compound can also be prepared in a form of ointment such as vaseline ointment.

When topically administered to skin, the compound represented by Formular I, a geometric isomer, a pharmaceutically acceptable salts and/or a solvate and/or a hydrate thereof can be prepared into a suitable form such as an ointment, a lotion or a cream, in which the active ingredient is suspended or dissolved in one or more carriers. The carrier for use in an ointment includes, but is not limited to mineral oil, liquid petrolatum, white petrolatum, propylene glycol, polyethylene oxide, polypropylene oxide, emulsifying wax, and water. The carrier for use in a lotion or a cream includes, but is not limited to mineral oil, sorbitan monostearate, Tween-60, cetyl ester wax, hexadecenyl aryl alcohol, 2-octyldodecanol, benzyl alcohol and water.

When topically administered to lower intestinal tract, the compound represented by Formular I, a geometric isomer, a pharmaceutically acceptable salt and/or a solvate and/or a hydrate thereof can be prepared into a form such as rectal suppository as described above or a suitable enema preparation form, in addition, a topical transdermal patch

can also be used.

The compound represented by Formular I, a geometric isomer, a pharmaceutically acceptable salt and/or a solvate and/or a hydrate thereof can also be administered in a preparation form of sterile injection, including sterile injectable aqueous solution or oil suspension, or sterile injectable solutions, wherein the usable carrier and solvent includes water, Ringer's solution and isotonic sodium chloride solution. In addition, a sterilized non-volatile oil such as monoglyceride or diglyceride can also be used as solvent or suspension media.

The drugs of the above various preparation forms can be prepared according to conventional methods in the pharmaceutical field.

In the present application, the term "therapeutically effective amount" or "prophylactically effective amount" refers to an amount that is sufficient to treat or prevent a patient's disease but is sufficiently low to avoid serious side effects (at a reasonable benefit/risk ratio) within a reasonable medical judgment. The therapeutically or prophylactically effective amount of the compound will change according to the factors such as the selected specific compound (e.g., considering the efficacy, effectiveness, and half-life of compound), the selected administration route, the disease to be treated or prevented, the severity of the disease to be treated or prevented, the treated or prevented patient's age, size, weight and physical disease, medical history, duration of treatment or prevention, nature of concurrent therapy, desired therapeutic or prophylactic effect, etc., but can still be routinely determined by those skilled in the art.

In addition, it should be noted that the specific dosage and method of using the compound represented by Formular I, a geometric isomer, a pharmaceutically acceptable salt and/or a solvate and/or a hydrate thereof for different patients depends on many factors, including the patient's age, weight, gender, natural health status, nutritional status, active strength of drug, administration time, metabolic rate, severity of disease, and subjective judgment of physician. Herein it is preferred to use a dosage between 0.0001-1000 mg/kg body weight/day.

**Brief Description of the Drawings**

Figure 1 shows that nitazoxanide can effectively reduce the viral nucleic acid load in Vero E6 cells infected by SARS-CoV-2. In Figure 1, (a) shows that nitazoxanide can reduce the viral RNA load in the cells 48 hours after the cells were infected by SARS-CoV-2. The ordinate is the copy number of viral RNA in the sample, and the abscissa is the drug concentration; (b) shows that the test cells were treated by nitazoxanide at the test concentration for 48 hours, and no cytotoxicity was observed. The ordinate is the percentage of cell viability relative to the vehicle control group (only cells, no drug was added), and the abscissa is the drug concentration.

**Specific Models for Carrying Out the Invention**

The following examples are illustrative preferred embodiments of the present application and do not constitute any limitation to the present application.

Example 1: Experiment on nitazoxanide reducing viral nucleic acid load in SARS-CoV-2 infected cells

(1) Drug treatment of virus-infected cells

Vero E6 cells (purchased from ATCC, Catalog No. 1586) was inoculated on a 24-well plate, cultured for 24 hours; then virus infection was carried out. Specifically, SARS-CoV-2 (2019-nCoV) virus (nCoV-2019BetaCoV/Wuhan/WIV04/2019 strain, provided by Wuhan Institute of Virology, Chinese Academy of Sciences) was diluted with 2% cell maintenance solution (formulation: FBS (purchased from Gibco company, Catalog No. 16000044) was added to MEM (purchased from Gibco, Catalog No. 10370021) at a volume ratio of 2%, thereby obtaining the 2% cell maintenance solution) to a corresponding concentration, and then added to a 24-well plate so that each well contained a viral load of 100$TCID_{50}$. Nitazoxanide (purchased from Selleck Chemicals, Catalog No. S1627) were diluted with 2% cell maintenance solution to corresponding concentrations and added separately to the corresponding wells, so that the final concentrations of the drugs were 100 $\mu$M, 33 $\mu$M, 11 $\mu$M, 3.7 $\mu$M, 1.23$\mu$M, 0.41$\mu$M, 0.14$\mu$M, respectively, then the plate was placed in a 37°C, 5% $CO_2$ incubator and cultured for 48 hours. To the vehicle control group, the 2% cell maintenance solution without any test drugs was added.

(2) RNA extraction

RNA extraction kit was purchased from Qiagen Company, Catalog No. 74106. The consumables (spin columns, RNase-free 2ml collection tubes, etc.) and reagents (RLT, RW1, RPE, RNase-free water, etc.) involved in the following RNA extraction steps were part of the kit. The following extraction steps were recommended steps in the kit instruction.

1) 100μL of the supernatant was taken from the tested plate and added to a nuclease-free EP tube, then 350μL of Buffer RLT was added to each well and mixed by beating with a transfer liquid gun until complete lysis was achieved, then centrifugation was carried out to obtain a supernatant;

2) an equal volume of 70% ethanol was added to the supernatant obtained in 1) and mixed well;

3) the mixture solution obtained in 2) was transferred to a RNase-free spin column, and centrifuged at 12000 rpm for 15 seconds, and the waste liquid was discarded;

4) 700μL of Buffer RW1 was added to the spin column, and centrifuged at 12000 rpm for 15 seconds to clean the spin column, and the waste liquid was discarded;

5) 500μL of Buffer RPE was added to the spin column, and centrifuged at 12000 rpm for 15 seconds to clean the spin column, and the waste liquid was discarded;

6) 500μL of Buffer RPE was added to the spin column, and centrifuged at 12000 rpm for 2min to clean the spin column, the waste was discarded;

7) a new RNase-free 2ml collection tube was used for replacement, centrifugation was carried out at 12000 rpm for 1 min, the spin column was dried, and then the spin column was transferred to a 1.5ml collection tube in step 8);

8) a new 1.5ml collection tube was used for replacement, in which the spin column dried in step 7) was placed, and 30μl of RNase-free water was added to the spin column, and centrifugation was carried out at 12000 rpm for 2 minutes, the obtained eluate contained the corresponding RNA, then the RNase inhibitor (purchased from NEB company, Catalog No. M0314L) was added, and Nano Drop (purchased from Thermo scientific, Nano Drop One) was used to detect each RNA concentration.

(3) RNA reverse transcription

In the experiment, the reverse transcription kit (PrimeScript™ RT reagent Kit with gDNA Eraser, Catalog No. RR047Q) produced by TaKaRa was used for RNA reverse transcription. The steps were as follows.

① Removal of gDNA: RNA samples of each experimental group were collected, 1 μg of each sample was taken for reverse transcription. First, 2μl of 5× gDNA Eraser Buffer was added to the RNA sample of each experimental group, the reaction system was supplemented with RNase-free water to reach 10μl, mixed well, and subjected to water bath at 42°C for 2 min to remove the gDNA that might be present in the sample;

② Reverse transcription: Appropriate amounts of enzyme, primer Mix and reaction buffer were added to the sample obtained in ①, RNase-free water was added to supplement to reach a volume of 20 μl, the reaction was performed in a water bath at 37°C for 15 minutes, and then in water bath at 85°C for 5 seconds, to obtain cDNA by transcription.

(4) Real-time PCR

Fluorescence quantitative PCR was used to detect the number of copies per milliliter of the original virus solution.

The reaction system was mixed by using TB Green Premix (Takara, Cat#RR820A), and the amplification reaction and reading were carried out with StepOne Plus Real-time PCR instrument (brand: ABI). The copy number contained in per milliliter of the original virus solution was calculated. The steps were as follows:

① Establishment of standard product: the plasmid pMT-RBD (the plasmid was provided by Wuhan Institute of Virology, Chinese Academy of Sciences) was diluted to $5\times10^8$ copies/μL, $5\times10^7$ copies/μL, $5\times10^6$ copies/μL, $5\times10^5$ copies/ μL, $5\times10^4$ copies/μL, $5\times10^3$ copies/μL, $5\times10^2$ copies/μL, respectively. 2 μL of standard product or cDNA template was taken for qPCR reaction.

② The primer sequences used in the experiment were as follows (all indicated in the 5'-3' direction):

RBD-qF: CAATGGTTTAACAGGCACAGG

RBD-qR: CTCAAGTGTCTGTGGATCACG

③ The reaction procedure was as follows:

Pre-denaturation: 95°C for 5 minutes;

Cycle parameters: 95°C for 15 seconds, 54°C for 15 seconds, 72°C for 30 seconds, a total of 40 cycles.

(5) Cytotoxicity test of drugs

The cytotoxicity test of drugs was carried out by using CCK-8 kit (Beyotime). Specific steps were as follows:

① $1\times10^4$ Vero-E6 cells (ATCC) were inoculated in a 96-well plate and cultured at 37°C for 8 hours.

② The drug was diluted with DMSO to an appropriate concentration of mother solution, and then diluted with MEM (purchased from Gibco company, Catalog No. 10370021) medium containing 2% FBS (purchased from Gibco company, Catalog No. 16000044) to the same concentration as that for the drug treatment. The original medium in the 96-well plate was discarded, 100 μL of the drug-containing MEM medium was taken and added to the cells, and three replicate wells were set for each concentration. A vehicle control (adding DMSO and medium to cells in wells, without adding drug) and a blank control (adding DMSO and medium to the wells, without cells) were set up. After the drug was added, the cells were cultured at 37°C for 48 hours.

③ 20 μL of CCK-8 solution (Beyotime) was added to the well to be tested, mixed gently without generating bubbles, and cultured subsequently at 37°C for 2 hours. $OD_{450}$ was read on a microplate reader (purchased from Molecular Devices, model: SpectraMax M5), and the cell viability was calculated:

$$\text{Cell viability (\%)} = (A_{(\text{drug treatment group})} - A_{(\text{blank control})}) / (A_{(\text{vehicle control})} - A_{(\text{blank control})}) \times 100\%$$

Wherein, A was the reading of the microplate reader.

(6) Experimental results

The results of the virus proliferation inhibition experiment showed that the test compound at concentrations of 100 μM, 33 μM, 11.1 μM and 3.7 μM could effectively inhibit the replication of SARS-CoV-2 virus genome in the infected supernatant (Table 1).

Table 1. *In vitro* antiviral test results of the test compound (nitazoxanide)

| Concentration (μM) | 100 | 33.33 | 11.11 | 3.70 | 1.23 | 0.41 | 0.14 | Vehicle |
|---|---|---|---|---|---|---|---|---|
| Copy number of virus genome | 1220444.5±130618.89 | 1424671.5±80107.4 2 | 5389366.5±187167 7.00 | 21027512.5± 1791348.26 | 76022948.5± 10886647.82 | 363337006± 85827045.67 | 820470664.5 ±130183808.09 | 74119457±846 3359.65 |

The cytotoxicity test results showed that the treatment of the test compound nitazoxanide did not change the cell viability at all test concentrations, that was, the test compound had no toxic effect on the cells at all concentrations (Table 2 and Figure 1).

Table 2. Cytotoxicity test results of the test compound (nitazoxanide)

| Concentration (μM) | 100 | 33.33 | 11.11 | 3.70 | 1.23 | 0.41 | 0.14 | Vehicle |
|---|---|---|---|---|---|---|---|---|
| Cell viability (% of vehicle control) | 93.29 ± 1.55 | 93.58 ± 0.75 | 96.03 ± 1.34 | 96.47 ± 3.10 | 98.54 ± 1.28 | 101.40 ±2.34 | 98.78± 2.38 | 103.47 ± 2.65 |

## Claims

1. Use of a compound represented by Formula I and/or Formular II, a geometric isomer, a pharmaceutically acceptable salt and/or a solvate and/or a hydrate thereof in the manufacture of a medicament for the prevention and/or the treatment of a disease or an infection caused by a coronavirus (such as SARS-CoV-2) (including but not limited to a respiratory disease (e.g., simple infection (such as fever, cough and sore throat), pneumonia, acute respiratory infection or severe acute respiratory infection, hypoxic respiratory failure and acute respiratory distress syndrome), sepsis and septic shock, etc.),

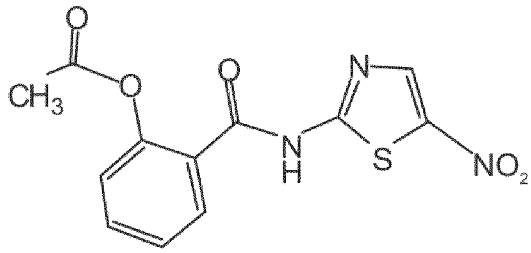

Formular I

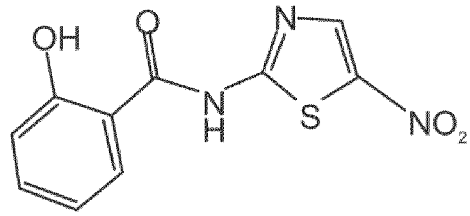

Formular II.

2. Use of a pharmaceutical composition in the manufacture of a medicament for the prevention and/or the treatment of a disease or an infection caused by a coronavirus (such as SARS-CoV-2) (e.g., a respiratory disease (e.g., simple infection (such as fever, cough and sore throat), pneumonia, acute respiratory infection or severe acute respiratory infection, hypoxic respiratory failure and acute respiratory distress syndrome), sepsis and septic shock, etc.), wherein the pharmaceutical composition comprises a compound represented by Formula I and/or Formular II, a geometric isomer, a pharmaceutically acceptable salt and/or a solvate and/or a hydrate thereof,

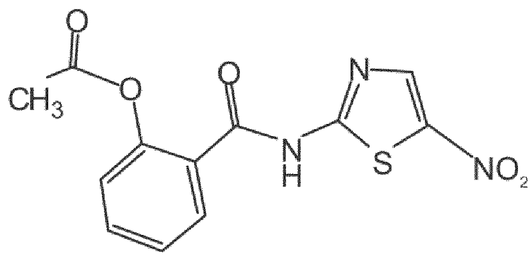

Formular I

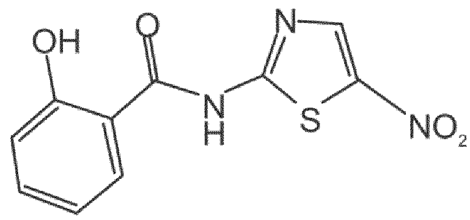

Formular II

preferably, wherein the pharmaceutical composition further comprises a pharmaceutically acceptable carrier or excipient; specifically, the pharmaceutical composition is a solid preparation, an injection, an external preparation, a spray, a liquid preparation, or a compound preparation.

3. Use of a compound represented by Formula I and/or Formular II, a geometric isomer, a pharmaceutically acceptable salt and/or a solvate and/or a hydrate thereof in the manufacture of a medicament as a coronavirus (such as SARS-CoV-2) inhibitor,

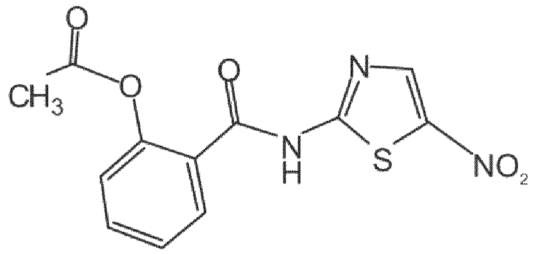

Formular I

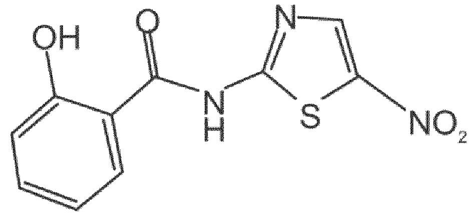

Formular II.

4. Use of a compound represented by Formula I and/or Formular II, a geometric isomer, a pharmaceutically acceptable salt and/or a solvate and/or a hydrate thereof in the manufacture of a medicament for inhibiting the replication or reproduction of coronavirus (such as SARS-CoV-2) in a cell (e.g., a cell of mammal),

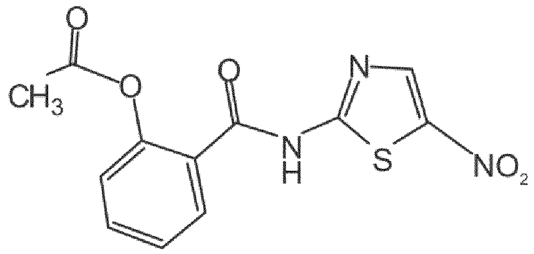

Formular I

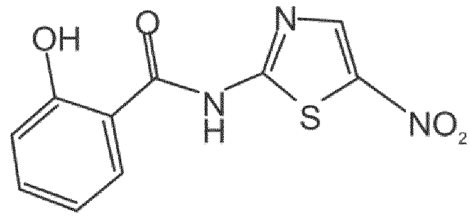

Formular II.

5. Use of a pharmaceutical composition in the manufacture of a medicament as a coronavirus (such as SARS-CoV-2) inhibitor,

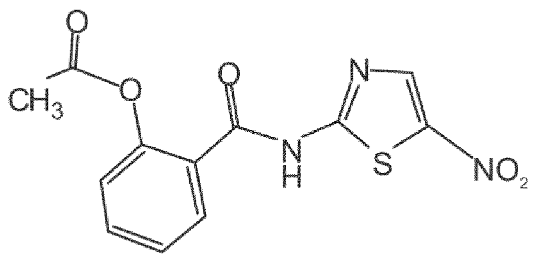

Formular I

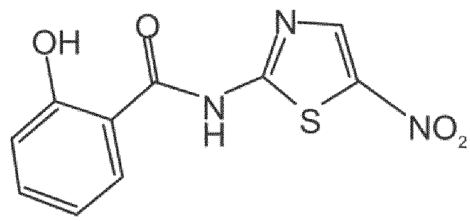

Formular II

wherein the pharmaceutical composition comprises a compound represented by Formula I and/or Formular II, a geometric isomer, a pharmaceutically acceptable salt and/or a solvate and/or a hydrate thereof,
preferably, wherein the pharmaceutical composition further comprises a pharmaceutically acceptable carrier or excipient; specifically, the pharmaceutical composition is a solid preparation, an injection, an external preparation, a spray, a liquid preparation, or a compound preparation.

6. Use of a pharmaceutical composition in the manufacture of a medicament for inhibiting the replication or reproduction of coronavirus (such as SARS-CoV-2) in a cell (e.g., a cell of mammal),

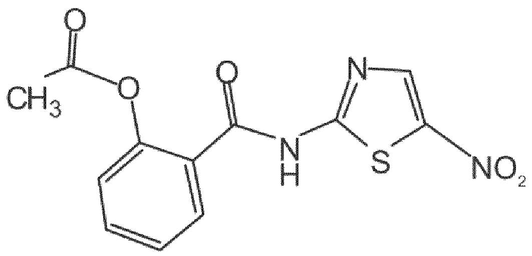

Formular I

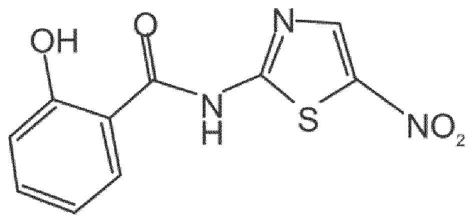

Formular II

wherein the pharmaceutical composition comprises a compound represented by Formula I and/or Formular II, a geometric isomer, a pharmaceutically acceptable salt and/or a solvate and/or a hydrate thereof, preferably, wherein the pharmaceutical composition further comprises a pharmaceutically acceptable carrier or excipient; specifically, the pharmaceutical composition is a solid preparation, an injection, an external preparation, a spray, a liquid preparation, or a compound preparation.

**7.** The use according to claim 1 or 2, wherein the disease caused by a SARS-CoV-2 is COVID-19.

**8.** The use according to claim 4 or 6, wherein the mammal includes bovine, equine, caprid, suidae, canine, feline, rodent, primate, for example, is human, cat, dog or pig.

**9.** A method for preventing and/or treating a disease in a mammal in need thereof, wherein the method comprises administering to the mammal in need thereof a therapeutically and/or prophylactically effective amount of a pharmaceutical composition comprising a compound represented by Formula I and/or Formular II, a geometric isomer, a pharmaceutically acceptable salt and/or a solvate and/or a hydrate thereof, or a compound represented by Formula I and/or Formular II, a geometric isomer, a pharmaceutically acceptable salt and/or a solvate and/or a hydrates thereof,

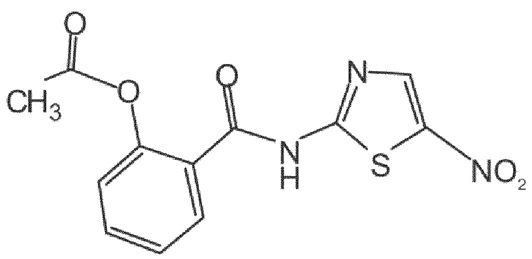

Formular I

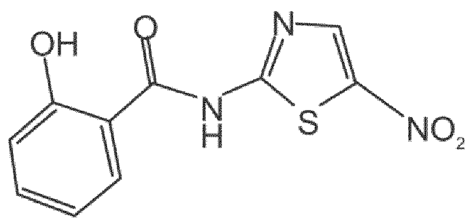

Formular II

wherein, the disease includes a disease or an infection caused by a coronavirus (such as SARS-CoV-2) (e.g., severe acute respiratory infection (SARI)).

**10.** The method according to claim 9, wherein the disease is a disease or an infection caused by a SARS-CoV-2.

**11.** The method according to claim 10, wherein the disease or the infection caused by a SARS-CoV-2 includes but not is limited to a respiratory disease (e.g., simple infection (such as fever, cough and sore throat), pneumonia, acute respiratory infection or severe acute respiratory infection, hypoxic respiratory failure and acute respiratory distress syndrome), sepsis and septic shock, etc.

**12.** A method for inhibiting the replication or reproduction of a coronavirus (such as SARS-CoV-2) in a mammal in need thereof, wherein the method comprises administering to the mammal in need thereof a pharmaceutical composition comprising a compound represented by Formula I and/or Formular II, a geometric isomer, a pharmaceutically acceptable salt and/or a solvate and/or a hydrate thereof, or a compound represented by Formula I and/or Formular II, a geometric isomer, a pharmaceutically acceptable salt and/or a solvate and/or a hydrates thereof,

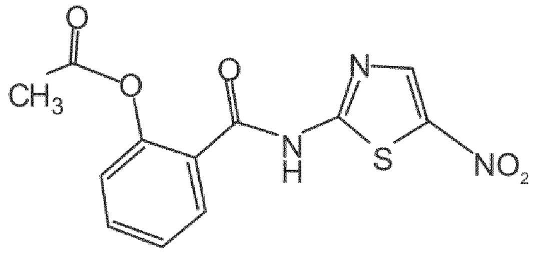

Formular I

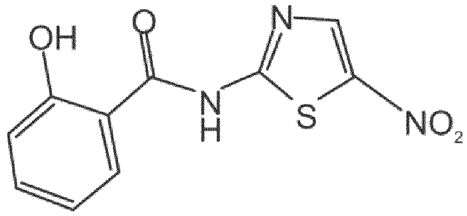

Formular II.

**13.** A compound represented by Formula I and/or Formular II, a geometric isomer, a pharmaceutically acceptable salt and/or a solvate and/or a hydrate thereof, for use in preventing and/or treating a disease or an infection caused by a coronavirus (such as SARS-CoV-2) (including but not limited to a respiratory disease (e.g., simple infection (such as fever, cough and sore throat), pneumonia, acute respiratory infection or severe acute respiratory infection, hypoxic respiratory failure and acute respiratory distress syndrome), sepsis and septic shock, etc.),

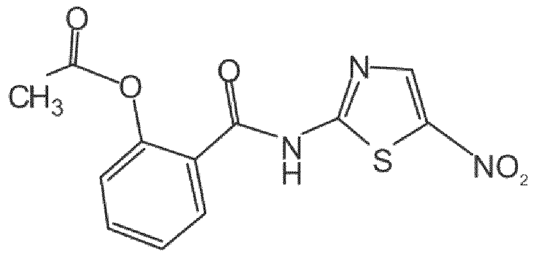

Formular I

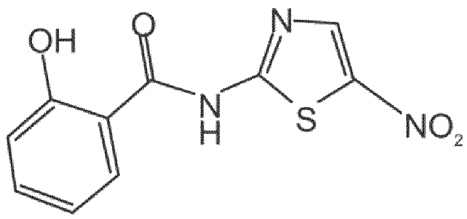

Formular II.

**14.** A compound represented by Formula I and/or Formular II, a geometric isomer, a pharmaceutically acceptable salt and/or a solvate and/or a hydrate thereof, for use as a coronavirus (such as SARS-CoV-2) inhibitor,

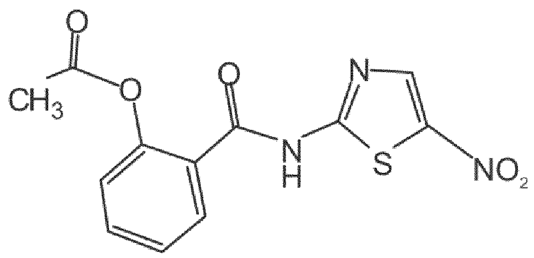

Formular I

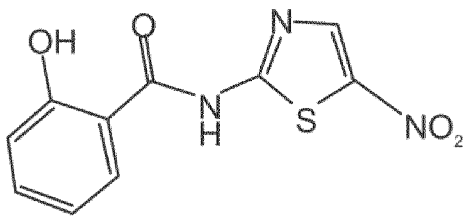

Formular II.

**15.** A compound represented by Formula I and/or Formular II, a geometric isomer, a pharmaceutically acceptable salt and/or a solvate and/or a hydrate thereof, for use in inhibiting the replication or reproduction of coronavirus (such as SARS-CoV-2) in a cell (e.g., a cell of mammal),

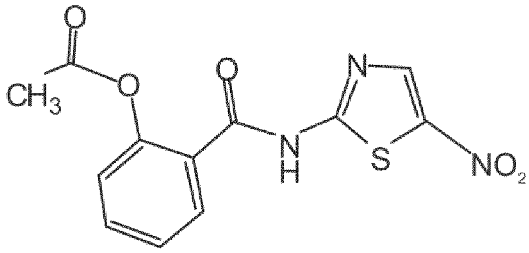

Formular I

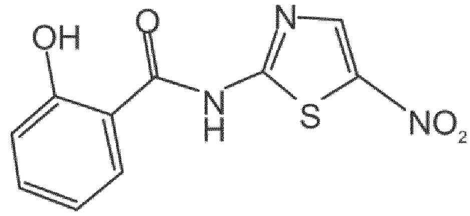

Formular II.

**16.** A pharmaceutical composition, for use in preventing and/or treating a disease or an infection caused by a coronavirus (such as SARS-CoV-2) (e.g., a respiratory disease (e.g., simple infection (such as fever, cough and sore throat), pneumonia, acute respiratory infection or severe acute respiratory infection, hypoxic respiratory failure and acute respiratory distress syndrome), sepsis and septic shock, etc.), wherein the pharmaceutical composition comprises a compound represented by Formula I and/or Formular II, a geometric isomer, a pharmaceutically acceptable salt and/or a solvate and/or a hydrate thereof,

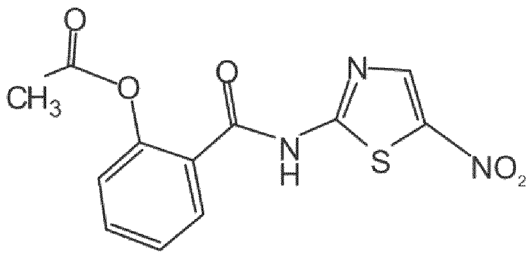

Formular I

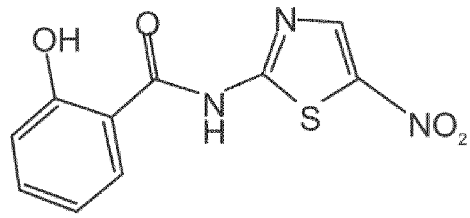

Formular II

preferably, wherein the pharmaceutical composition further comprises a pharmaceutically acceptable carrier or excipient; specifically, the pharmaceutical composition is a solid preparation, an injection, an external preparation, a spray, a liquid preparation, or a compound preparation.

**17.** A pharmaceutical composition, for use as a coronavirus (such as SARS-CoV-2) inhibitor, wherein the pharmaceutical composition comprises a compound represented by Formula I and/or Formular II, a geometric isomer, a pharmaceutically acceptable salt and/or a solvate and/or a hydrate thereof,

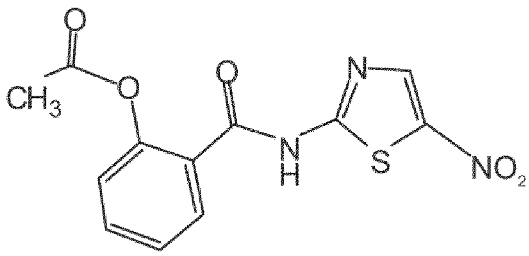

Formular I

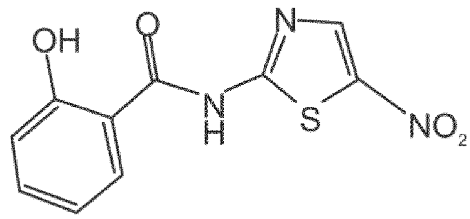

Formular II

preferably, wherein the pharmaceutical composition further comprises a pharmaceutically acceptable carrier or excipient; specifically, the pharmaceutical composition is a solid preparation, an injection, an external preparation, a spray, a liquid preparation, or a compound preparation.

**18.** A pharmaceutical composition, for use in inhibiting the replication or reproduction of coronavirus (such as SARS-CoV-2) in a cell (e.g., a cell of mammal), wherein the pharmaceutical composition comprises a compound represented by Formula I and/or Formular II, a geometric isomer, a pharmaceutically acceptable salt and/or a solvate and/or a

hydrate thereof,

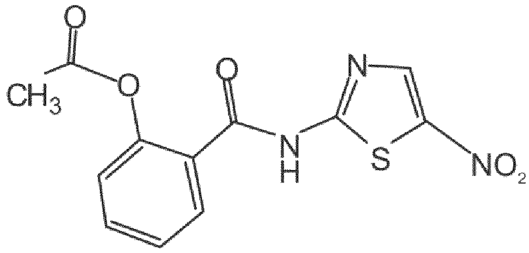

Formular I

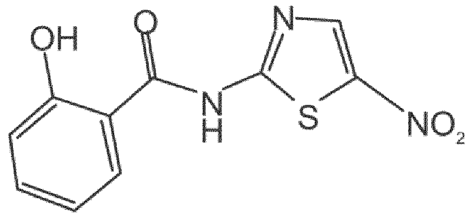

Formular II

preferably, wherein the pharmaceutical composition further comprises a pharmaceutically acceptable carrier or excipient; specifically, the pharmaceutical composition is a solid preparation, an injection, an external preparation, a spray, a liquid preparation, or a compound preparation.

**19.** The method according to claim 10, the compound, a geometric isomer, a pharmaceutically acceptable salt and/or a solvate and/or a hydrate thereof according to claim 13, or the composition according to claim 16, wherein the disease caused by a SARS-CoV-2 is COVID-19.

**20.** The method according to anyone of claims 9 to 12, or the compound, a geometric isomer, a pharmaceutically acceptable salt and/or a solvate and/or a hydrate thereof according to claim 15, or the composition according to claim 18, wherein the mammal includes bovine, equine, caprid, suidae, canine, feline, rodent, primate, wherein the preferred mammal is a human, a cat, a dog, or a pig.

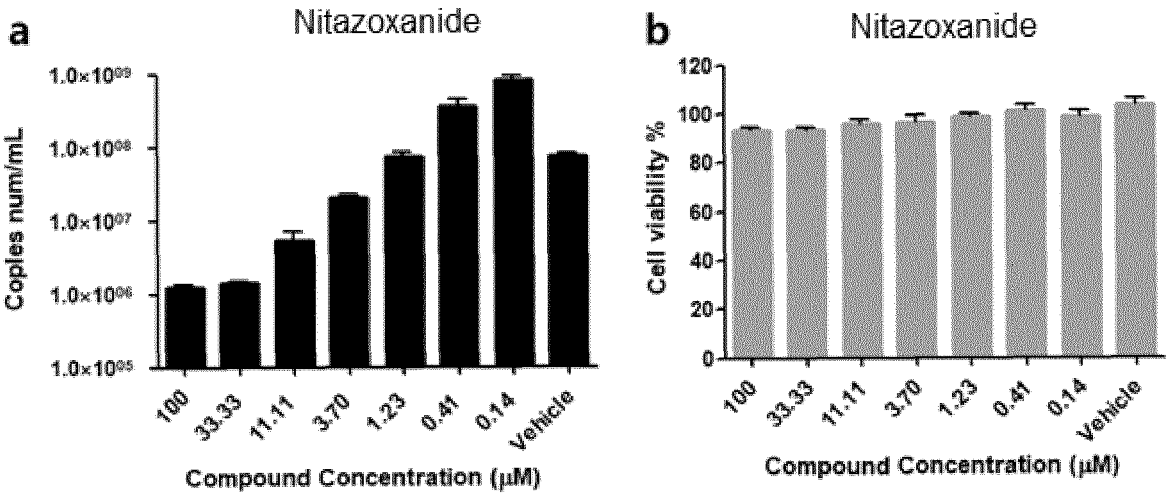
a
Nitazoxanide
Copies num/mL
1.0×10^09
1.0×10^08
1.0×10^07
1.0×10^06
1.0×10^05
100
33.33
11.11
3.70
1.23
0.41
0.14
Vehicle
Compound Concentration (μM)
b
Nitazoxanide
Cell viability %
120
100
80
60
40
20
0
100
33.33
11.11
3.70
1.23
0.41
0.14
Vehicle
Compound Concentration (μM)

Figure 1

**INTERNATIONAL SEARCH REPORT**

International application No.
**PCT/CN2020/102143**

**A. CLASSIFICATION OF SUBJECT MATTER**

A61K 31/426(2006.01)i； A61P 31/12(2006.01)i； A61P 31/14(2006.01)i

According to International Patent Classification (IPC) or to both national classification and IPC

**B. FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNABS; CNTXT; DWPI; SIPOABS; USTXT; EPTXT; WOTXT; STNext: 中国人民解放军军事科学院军事医学研究院; 钟武; 硝唑尼特; 替唑尼特; 冠状病毒; Nitazoxanide; coronavirus; SARS-Cov-2; MERS-CoV

**C. DOCUMENTS CONSIDERED TO BE RELEVANT**

| Category* | Citation of document, with indication, where appropriate, of the relevant passages | Relevant to claim No. |
|---|---|---|
| X | CN 105853415 A (ROMARK LABORATORIES, L.C.) 17 August 2016 (2016-08-17) claims 1-4 and 6; description, paragraphs [0643] and [0644] | 1-20 |
| X | CN 108042535 A (ROMARK LABORATORIES, L.C.) 18 May 2018 (2018-05-18) claims 53-58, 67, 69 and 76 | 1-20 |
| X | ROSSIGNOL J. F. "Nitazoxanide, a New Drug Candidate for the Treatment of Middle East Respiratory Syndrome Coronavirus;" *Journal of Infection and Public Health;*, Vol. 9, 31 December 2016 (2016-12-31), pp. 227-230 | 1-20 |
| X | ROSSIGNOL J. F. "Nitazoxanide: A First-in-Class Broad-Spectrum Antiviral Agent;" *Antiviral Research,* Vol. 110, 07 August 2014 (2014-08-07), pp. 94-103 | 1-20 |
| X | CAO J. Z. et al. "A Screen of the NIH Clinical Collection Small Molecule Library Identifies Potential Anti-Coronavirus Drugs； " *Antiviral Research,* Vol. 114, 28 November 2014 (2014-11-28), pp. 1-10 | 1-20 |

☐ Further documents are listed in the continuation of Box C.　☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **10 October 2020** | **29 October 2020** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/102143**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**

International application No.
**PCT/CN2020/102143**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **9-12,19-20**
because they relate to subject matter not required to be searched by this Authority, namely:

[1] Claims 9-12, 19 and 20 relate to a method for prevention and/or disease in mammals in need, which do not comply with PCT Rule 39.1(iv). A search is made on the basis of the subject name being the use of compounds, or their geometric isomers, or pharmaceutically acceptable salts, or their solubilization compounds, or the pharmaceutical compositions of their hydrates shown in formula I and/or formula II in the preparation of drugs for treatment or prevention of diseases or infection induced by coronavirus.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2020/102143**

| Patent document cited in search report | Publication date (day/month/year) | Patent family member(s) | Publication date (day/month/year) |
|---|---|---|---|
| CN 105853415 A | 17 August 2016 | EA 021587 B1 | 30 July 2015 |
| | | AP 201106015 A0 | 31 December 2011 |
| | | AP 3074 A | 31 December 2014 |
| | | US 8846727 B2 | 30 September 2014 |
| | | US 2010292274 A1 | 18 November 2010 |
| | | WO 2010132404 A1 | 18 November 2010 |
| | | KR 20120036827 A | 18 April 2012 |
| | | HK 1178512 A1 | 28 July 2017 |
| | | BR PI1010547 A2 | 09 April 2019 |
| | | EP 2429986 A4 | 14 November 2012 |
| | | CA 2761876 C | 03 January 2017 |
| | | HN 2011003002 A | 03 August 2015 |
| | | IL 240167 D0 | 24 September 2015 |
| | | CA 2761876 A1 | 18 November 2010 |
| | | CN 102803203 A | 28 November 2012 |
| | | SG 176045 A1 | 29 December 2011 |
| | | AU 2016202439 A1 | 12 May 2016 |
| | | JP 2016006053 A | 14 January 2016 |
| | | EA 201171378 A1 | 30 May 2012 |
| | | CO 6460766 A2 | 15 June 2012 |
| | | MX 337460 B | 03 March 2016 |
| | | CN 102803203 B | 11 May 2016 |
| | | US RE47786 E | 31 December 2019 |
| | | IL 216331 A | 28 June 2018 |
| | | NZ 596538 A | 30 April 2014 |
| | | DO P2011000353 A | 31 July 2012 |
| | | EC SP11011511 A | 30 March 2012 |
| | | PE 20121118 A1 | 05 September 2012 |
| | | CL 2011002830 A1 | 23 March 2012 |
| | | EP 2429986 B1 | 16 November 2016 |
| | | US 2014341850 A1 | 20 November 2014 |
| | | IL 240167 A | 24 September 2015 |
| | | EP 3078377 A2 | 12 October 2016 |
| | | SG 10201402281W A | 30 July 2014 |
| | | CR 20110607 A | 13 February 2012 |
| | | AU 2016202439 B2 | 15 June 2017 |
| | | US RE46724 E | 20 February 2018 |
| | | NI 201100199 A | 28 March 2012 |
| | | AU 2010247816 A1 | 01 December 2011 |
| | | EP 3078377 A3 | 05 April 2017 |
| | | US 9126992 B2 | 08 September 2015 |
| | | IL 216331 D0 | 31 January 2012 |
| | | KR 101760956 B1 | 24 July 2017 |
| | | HK 1223819 A1 | 11 August 2017 |
| | | AP 201106015 D0 | 31 December 2011 |
| | | JP 5918124 B2 | 18 May 2016 |
| | | JP 6193922 B2 | 06 September 2017 |
| | | AP 2011006015 A0 | 31 December 2011 |
| | | EP 2429986 A1 | 21 March 2012 |
| CN 108042535 A | 18 May 2018 | US 2010330173 A1 | 30 December 2010 |

Form PCT/ISA/210 (patent family annex) (January 2015)

**INTERNATIONAL SEARCH REPORT**
**Information on patent family members**

International application No.
**PCT/CN2020/102143**

| Patent document cited in search report | Publication date (day/month/year) | Patent family member(s) | | | Publication date (day/month/year) |
|---|---|---|---|---|---|
| | | JP | 2017197552 | A | 02 November 2017 |
| | | JP | 2016172749 | A | 29 September 2016 |
| | | EA | 201270077 | A1 | 28 December 2012 |
| | | KR | 101812054 | B1 | 27 December 2017 |
| | | MX | 2012000230 | A | 25 January 2012 |
| | | JP | 5932640 | B2 | 08 June 2016 |
| | | US | 2019307730 | A1 | 10 October 2019 |
| | | EP | 2445349 | A1 | 02 May 2012 |
| | | AP | 201206064 | D0 | 29 February 2012 |
| | | ZA | 201200263 | B | 26 September 2012 |
| | | AU | 2017221828 | A1 | 21 September 2017 |
| | | CA | 2968113 | C | 14 May 2019 |
| | | WO | 2010151577 | A1 | 29 December 2010 |
| | | AU | 2010264479 | A1 | 19 January 2012 |
| | | US | 9820975 | B2 | 21 November 2017 |
| | | US | 9023877 | B2 | 05 May 2015 |
| | | CA | 2766642 | C | 05 September 2017 |
| | | CA | 2766642 | A1 | 29 December 2010 |
| | | KR | 20170141830 | A | 26 December 2017 |
| | | AP | 2012006064 | A0 | 29 February 2012 |
| | | US | 2018078533 | A1 | 22 March 2018 |
| | | US | 2016228415 | A1 | 11 August 2016 |
| | | CN | 102480967 | A | 30 May 2012 |
| | | KR | 20180032689 | A | 30 March 2018 |
| | | JP | 6188863 | B2 | 30 August 2017 |
| | | CA | 2968113 | A1 | 29 December 2010 |
| | | US | 9345690 | B2 | 24 May 2016 |
| | | US | 2015250768 | A1 | 10 September 2015 |
| | | KR | 20120102570 | A | 18 September 2012 |
| | | HK | 1255283 | A1 | 09 August 2019 |
| | | BR | PI1014322 | A2 | 25 August 2015 |
| | | JP | 2012531420 | A | 10 December 2012 |
| | | KR | 20190120436 | A | 23 October 2019 |
| | | EA | 030679 | B1 | 28 September 2018 |
| | | US | 10363243 | B2 | 30 July 2019 |
| | | MX | 341877 | B | 06 September 2016 |
| | | AU | 2010264479 | B2 | 01 June 2017 |
| | | EP | 2445349 | A4 | 05 December 2012 |
| | | JP | 6464225 | B2 | 06 February 2019 |
| | | AP | 201206064 | A0 | 29 February 2012 |
| | | CA | 3038405 | A1 | 29 December 2010 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202010071688


### Non-patent literature cited in the description


- **ROSSIGNOL, J.F. ; CAVIER, R.** Synthesis and antiparasitic activity of 2-benzamidonitrothiazoles. *Chem. Abstr.,* 1975, vol. 83, 28216n
- **ROSSIGNOL JF.** Nitazoxanide: a first-in-class broad-spectrum antiviral agent. *Antiviral Res.,* 2014, vol. 110, 94-103
- **HAFFIZULLA J ; HARTMAN A ; HOPPERS M et al.** Effect of nitazoxanide in adults and adolescents with acute uncomplicated influenza: a double-blind, randomised, placebo-controlled, phase 2b/3 trial. *Lancet Infect Dis.,* 2014, vol. 14 (7), 609-18